# EUROPEAN PATENT SPECIFICATION

(11) **EP 3 494 865 B1**
(45) Date of publication and mention of the grant of the patent: **04.01.2023**
(21) Application number: 18207015.1
(22) Date of filing: 19.11.2018
(51) Int. Cl.: A61B 1/00, A61B 17/22, A61B 17/32, A61B 1/018

(54) **MEDICAL PROBE OR ENDOSCOPE WITH A COATING OF DIAMOND-LIKE CARBON**
MEDIZINISCHE SONDE ODER ENDOSKOP MIT EINER BESCHICHTUNG AUS DIAMANTARTIGEM KOHLENSTOFF
SONDE MÉDICALE OU ENDOSCOPE AVEC UN REVÊTEMENT DE CARBONE DE TYPE DIAMANT

(30) Priority: 17.11.2017 US 201762588277 P; 16.11.2018 US 201816193754
(43) Date of publication of application: 12.06.2019
(73) Proprietor: Gyrus ACMI, Inc., Southborough, MA 01772 (US)
(72) Inventor: BAKER, Charles Alan, Rogers, MN 55374 (US); PATEL, Rahul, Erie, PA 16506 (US)
(74) Representative: Haseltine Lake Kempner LLP

(56) References cited:
- WO-A1-2016/205335
- US-A1- 2006 150 862
- US-A1- 2009 246 243
- US-A1- 2016 066 975
- US-B1- 6 761 736

## Description

### TECHNICHAL FIELD

The technical field of the present disclosure is generally directed to medical probes. In particular, the medical probes disclosed herein are coated with a non-reflective coating.

### BRIEF SUMMARY

The present application is directed to a medical probe having at least an outer surface that is at least partially coated with a diamond-like carbon (DLC) material or a derivative of a DLC material. Medical probes coated with such a material are highly resistant to the loss of material from the coated surface. Providing the coating on the working tip of the probe has also been found to decrease wear, and thereby to increase the working life of the probe. In addition, because the coating has low reflectivity, the use of coated probes of the present application improves visibility in the working field of view. Diamond-like carbon coatings are known from US 2016/066975 and WO 2016/205335.

The scope of the present invention is defined by the claims which follow.

### BRIEF DESCRIPTION OF DRAWINGS

FIG. 1A is a side view of an endoscope.
FIG. 1B is a cross-sectional view of an endoscope taken along lines A - A of Fig. 1A.
FIG. 2 is an elevated perspective side view of the endoscope of Figs. 1A-2B.
FIG. 3 is an enlarged view of area C of Fig. 2.
FIG. 4 is a side view of a medical probe.
FIG. 5 is an elevated perspective side view of a medical probe.

### DETAILED DESCRIPTION

As used herein, endoscopy refers generally to a medical procedure in which an instrument is introduced into a body in order to allow visualization of internal conditions. While an endoscopy procedure may be limited to such visualization, many endoscopy procedures also provide for treatment of certain conditions.

The medical probe described herein may be primarily used in the context of endoscopic procedures, including ultrasound lithotripsy. However, the probe may be used in any medical procedure with which the probe is physically and functionally compatible, and in which the probe's benefits of decreased loss of material from the outer surface of the probe, decreased wear of the working tip of the probe, and/or low reflectivity of the outer surface of the probe would be advantageous.

All references herein to the "patient" include both human and non-human patients, while references to the "operator" performing a procedure include a human co-located with the patient at the time of the procedure; a human located remote from the patient at the time of the procedure and operating the medical equipment remotely; and, an artificial intelligence or other advanced computing system performing a procedure autonomously, with or without with human oversight.

In general, lithotripsy refers to a class of procedures to reduce the size of hard masses in the biliary and/or urinary systems; for example, a patient's bladder, kidney, or ureter. The term "hard masses" as used herein includes but is not limited to solid formations of urates, oxalates and phosphates, such as gallstones, kidney stones, and cystine stones, present in the ducts and cavities of a living body.

Lithotripsy procedures may be broadly divided into extracorporeal lithotripsy, which is a non-invasive procedure in which energy is projected against the outer surface of the patient's body, and intracorporeal lithotripsy, in which an instrument is introduced into the body of the patient and energy is transmitted through the instrument to the interior of the body.

Intracorporeal lithotripsy may direct energy to the target hard masses in the form of, e.g., laser energy, mechanical energy, electrohydraulic energy, electro-impulse destruction and/or vibrational energy, particularly ultrasound.

Intracorporeal lithotripsy in which ultrasound is used to break up hardened masses, whether alone or in combination with other forms of energy, is referred to herein as "ultrasound intracorporeal lithotripsy" or, for brevity, simply as "ultrasound lithotripsy" or "ultrasonic lithotripsy".

The following table provides a representative, non-limiting list of types of endoscopes, the interior body areas they are used to visualize, and the name of the associated procedure or procedures performed with each type:

| Type | Area Visualized | Name of Procedure(s) |
|---|---|---|
| Arthroscope | Joints | Arthroscopy |
| Bronchoscope | Trachea and bronchi | Bronchoscopy |
| Colonoscope | Colon and large intestine | Colonoscopy, lower endoscopy |
| Cystoscope | Bladder | Cystoscopy, cystourethroscopy |
| Enteroscope | Small intestine | Enteroscopy |
| Esophagogastro-duodenoscope | Esophagus, stomach, and duodenum | Esophagogastro-duodenoscopy, upper endoscopy, panendoscopy, gastroscopy |
| Hysteroscope | Inside of uterus | Hysteroscopy |
| Laparoscope | Space inside abdomen and pelvis | Laparoscopy, peritoneal endoscopy |
| Laryngoscope | Larynx | Laryngoscopy |
| Mediastinoscope | Mediastinum (space between the lungs) | Mediastinoscopy |
| Sigmoidoscope | Rectum and sigmoid colon | Sigmoidoscopy, proctosigmoidoscopy |
| Thoracoscope | Space between lungs and chest wall | Thoracoscopy, pleuroscopy |
| Nephroscope | Kidney | |
| | Renal collection system: | Percutaneous Nephrolithotomy (PCNL) |
| Ureteroscope | Bladder/ureter/kidney | Ureteroscopy (URS) |

An additional type of endoscopic procedure is lithotripsy, in which a medical probe is used to transmit energy into the body in order to break up hardened masses in, most commonly, the kidney, bladder, or ureter. In such procedures, one end of the medical probe is connected to an energy source. The opposite end, which includes a working tip, is introduced into the body of the patient to bring the working tip into proximity or contact with the hardened masses. The energy source is then used to transmit energy from the energy source along the medical probe to the working tip. The energy is used to break the hardened masses into smaller pieces, which may be suctioned out, and/or naturally expelled or excreted by the patient.

One particular type of lithotripsy is ultrasonic lithotripsy. The medical probes used in ultrasonic lithotripsy may be single, in which the probe is a single elongated piece including an inner annulus for suctioning away the fragmented pieces of hardened mass. Alternately, the medical probe may be a multiple probe that is surrounded by one or more tubes, usually coaxial and at least substantially coextensive with the inner probe, and usually with a gap present between the inner surface of the outer tube and the outer surface of the probe. In this context, "tube" is used to mean an elongated structure having a hollow center or annulus; a single probe may therefore also be a tube. A surrounding tube may itself be a medical (such as an ultrasonic) probe, in which case the inner and outer probes may be used to transmit the same or different ultrasonic frequencies, with the use of different frequencies having the capability to improve the ability to fragment and remove hardened masses. Alternatively, the inner and outer probes may be used to transmit different types of energy, such as ultrasonic vibrations and mechanical shock.

Ultrasonic lithotripsy probes may be single, in which the probe is a single elongated piece which may have an inner annulus for suctioning away the fragmented pieces of hardened mass, or multiple. One example of a multiple ultrasonic lithotripsy probe is a dual probe. The surrounding tube in a dual probe design may also be used to transmit mechanical energy to the hardened mass, such as shock waves, and may itself be enclosed in another surrounding tube. In addition, an ultrasonic lithotripsy probe may be used in combination with shock waves provided by a free mass. The use of ultrasonic vibrations may also be combined with sonic vibrations.

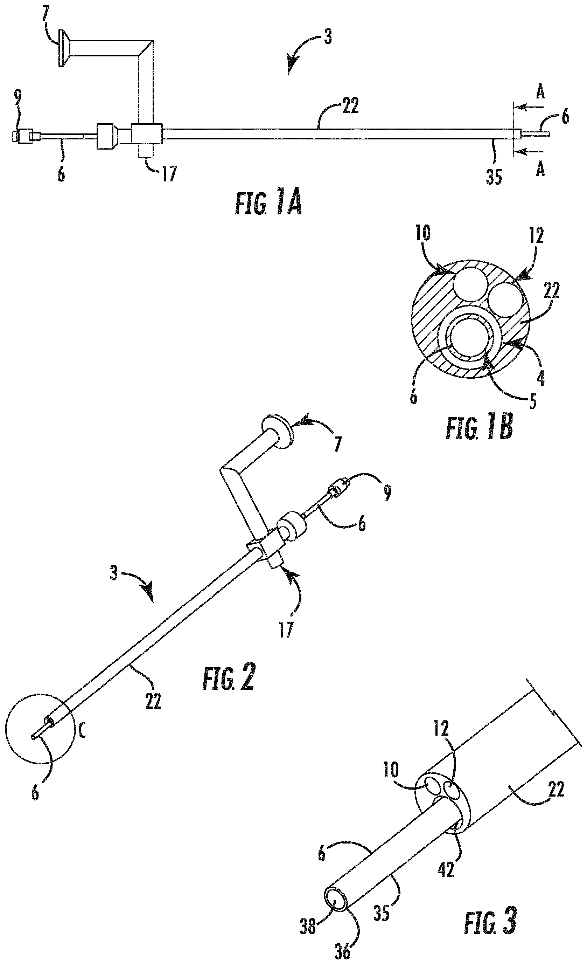

With reference to Figs. 1A-3, one embodiment of an endoscope 3 is shown. Endoscope 3 generally includes a first end 20 and a second end 25. An insertion rod 22 is provided for introduction into an intracavitary region of a patient at distal end 35. A medical probe 6 extends through endoscope body 22. An endoscope eye piece 7 and endoscope light source connection 17 are also provided. A fitting 9 is included at a second end for coupling to an endoscopic apparatus, such as an ultrasonic transducer.

As shown in cross-sectional and enlarged views in Figs. 1B and 3, respectively, medical or endoscopy probe 6 extends out of endoscopy body 22. Fig. 1B is a cross-sectional view of endoscope 3 taken along lines A - A of Fig. 1 and shows an endoscope light source 10 and an endoscope viewing lens 12, through which the operator receives the output of the endoscope viewing lens. An endoscope instrument and irrigation lumen 4 and an endoscopy probe and aspiration lumen 5 are also shown in Fig. 1B, along with endoscopy probe 6.

In the embodiment shown, endoscopy probe 6 is surrounded directly by endoscope body 22. This configuration may result direct physical contact between the outer surface of endoscopy probe 6, and the inner surface of the endoscope body 22. In, particularly, the presence of at least the ultrasonic vibrations transmitted through endoscopy probe 6 by the ultrasonic transducer of the ultrasonic lithotripter system may cause physical interaction between the outer surface of endoscopy probe 6 and the inner surface of the endoscope body 22 that results in wear of and/or release of particles from at least the outer surface of the endoscopy probe 6.

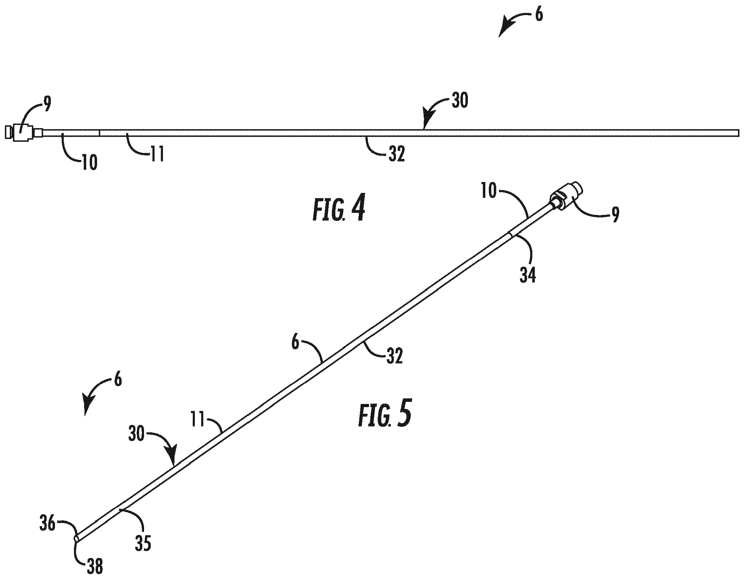

Figs. 4 and 5 are, respectively, side perspective and elevated side perspective views of medical probe 6 showing fitting 9, uncoated medical probe portion 30, and coated medical probe portion 32.

Medical probe 6 includes a probe body 30 extending along a longitudinal axis. The probe body 30 includes an exterior surface 32, a proximal portion 34, and a distal portion 35 including a distal tip 36. In particular, the proximal portion 34 includes a proximal end portion including fitting 9 adapted for connection to an endoscopy instrument. The distal portion 35 is adapted for insertion into the body of a patient.

Probe body 30 may be comprised of any material that is suitable for use in endoscopic or other medical procedures and capable of being coated with diamond-like carbon or diamond-like carbon derivative materials. Stainless steel may be used for medical probe bodies. One particular suitable type of stainless steel which may be used is grade 304 stainless steel.

At least a portion of the exterior surface of the distal portion 35 has a coating of diamond-like carbon or diamond-like carbon derivative, as discussed in more detail below. The medical probe 6 may also include an interior surface 38, as shown in Fig. 3. At least the distal tip 36 of medical probe 6 may have a coating of diamond-like carbon or of a diamond-like carbon derivative. Substantially the entire exterior surface 32 of the medical probe body 30 except for a proximal end portion 34 may have a coating of diamond-like carbon or diamond-like carbon derivative. In some embodiments, wherein endoscope body 30 includes an exterior surface 32 and an interior surface 38, at least a portion of the interior surface has a coating of diamond-like carbon or diamond-like carbon derivative. The medical probe body 32 may be comprised of austenitic stainless steel, such as grade 304 stainless steel. The coating of diamond-like carbon or diamond-like carbon derivative may have a thickness of from about 1.0 micrometers to about 6.0 micrometers, or of from about 2.0 micrometers to about 4.0 micrometers. The diamond-like carbon or diamond-like carbon derivative may include hydrogenated amorphous carbon, a metal, a metalloid, or combinations thereof. In one embodiment may include a-C:H: Si. As discussed supra, the medical probe may be an ultrasonic lithotripsy probe that includes a coating as described. The ultrasonic lithotripsy apparatus includes a source of ultrasonic vibration adapted for connection to the proximal end portion of the probe.

Materials used for coating the medical probe are comprised of hard amorphous carbon, and fall into the general class of diamond-like carbon (DLC). For purposes of the instant disclosure DLC is defined as metal-free amorphous carbon which may or may not include hydrogen. These DLC materials may be designated as either "a-C" or "a-C:H", where "a" is "amorphous", "a-C" is "amorphous carbon", and "a-C:H" is "hydrogenated amorphous carbon".

In certain embodiments, the DLC may be combined or doped with metals or metalloids. The resulting materials are, for purposes of this disclosure, defined as derivatives of DLC, with the metal or metalloid component represented by "Me". These DLC derivatives may therefore be designated generally as "DLC-Me" or "Me-DLC "; and more specifically as "Me-a-C" or "a-C:Me" for the non-hydrogenated form, and by "Me-a-C:H" or "a-C:H:Me" for the hydrogenated form.

When "Me" represents a metal, tungsten is commonly used, and may then be represented by its periodic table symbol of "W" rather than as "Me": for example, a DLC derivative consisting of non-hydrogenated amorphous carbon containing tungsten may be designated as "a-C:W". When "Me" represents a metalloid, silicon is commonly used, and the resulting DLC derivative may then be represented by its periodic table symbol of "Si" rather than as "Me". In this instance, a hydrogenated amorphous carbon containing silicon may be designated as "a-C:H:Si".

DLC and DLC derivative materials may be coated on surfaces using vapor deposition techniques well known in the art, such as physical vapor deposition (PVD) including sputtering, enhanced sputtering, ion plating, and arc evaporation; chemical vapor deposition (CVD); plasma-assisted chemical vapor deposition, which may also be called plasma-aided or plasma-enhanced vapor deposition (PACVD or PAVD); or a combination of PVD and PACVD or PAVD.

To produce the coated medical probe, a probe is coated with a DLC or DLC-Me material to produce a coating of from about 0.1 micrometers up to about 2.0 micrometers, or up to about 3.0 micrometers, or up to about 4.0 micrometers, or up to about 5.0 micrometers, or up to about 6.0 micrometers in thickness. The coating may be from about 2.0 micrometers to about 4.0 micrometers in thickness.

The coating material may have a microhardness (HV 0.05) of from about 2,000 up to at least about 2,400 or up to at least about 2,500; a coefficient of friction (dry against steel) of from about 0.05 or about 0.1 up to about 0.2; a maximum service temperature of at least about 300 °C; a coating temperature of from about 180 °C up to about 220 °C; and a coating color of charcoal or black. The coating material may be a DLC-Me material of the type a-C:H:Si. One example of such a coating material is Balinit^{®} Dylyn coating, available from Oerlikon Balzers (1475 E. Woodfield Rd., Suite 201, Schaumburg, IL 60173, USA).

The process of coating the medical probe or portion thereof may be carried out using conventional vapor deposition techniques, as previously stated herein. Prior to coating, the medical probe surface should be cleaned to remove contaminants that might otherwise interfere with the vapor deposition, for example by ultrasonic cleaning followed by wiping the medical probe with isopropyl alcohol.

It may be desirable to avoid coating some portion of medical probe 6, resulting in a medical probe with an uncoated portion 10 and a coated portion 11. For example, the proximal end 34 of the probe body 30 adapted for connection to the endoscopy instrument may provide conductivity for electrical, heat, or other energy that would be impaired by the coating. Alternatively, connecting or assembling the proximal end 34 of the medical probe adapted for connection to the endoscopy instrument for use may require welding, brazing, or similar techniques for creating a metal-to-metal connection that may be impaired by the presence of the coating. The portion 10 of medical probe 6 that is desired not to receive the coating may therefore be excluded from coating, such as by remaining outside the effective field where coating will occur, or by being physically shielded or covered during the coating process, which may be accomplished, for example, by use of an appropriate fixture that holds the medical probe during the vapor deposition process in such a manner that the coating will not reach the portion of the medical probe that is desired to remain free of the coating, resulting in medical probe 6 having an uncoated portion 10 and a coated portion 11. Alternatively, the entire medical probe may be coated, and the coating may then be removed from any portion of the medical probe where its presence is not desired.

Coated medical probes in accordance with the present disclosure have been found to comply with ISO 10993 of the International Standards Organization, "Biological evaluation of medical devices", and to be compatible with ethylene oxide sterilization.

When used in endoscopic procedures, a coated medical probe as taught herein will, in comparison to the same probe without a coating, exhibit higher resistance to stress and less surface wear from loss of the material of the probe's outer surface caused by the effects of the endoscopic energy passing along the probe, as well as less surface wear and resulting particles or other loose material from the inner surface of the endoscope. In the case of dual or other multiple medical probe systems, a coated medical probe as taught herein will, in comparison to the same probe without a coating, exhibit less surface wear from physical contact between the outer surface of an inner probe and the inner surface of an adjacent outer probe. In both instances, the clarity of the field of view for the medical procedure, such as may be provided through endoscope viewing lens 2, will be improved due to the lower incidence of loose probe material; and, the clarity of the field of view may be improved by the low reflectivity of the coating as compared to an uncoated probe whose outer surface material, such as stainless steel, has higher reflectivity than the coating. The presence of the coating taught herein on the working distal tip 36 of a medical probe will reduce wear on the working tip caused by the effects of endoscopic energy and/or abrasive wear resulting from physical contact of the working tip with hardened masses, improving the efficiency and/or useful life of the medical probe.

An interior surface 42 of an endoscope body may similarly be coated with a DLC or DLC-Me material following the teachings herein. An endoscope body 22 with a coated interior surface may be used in combination with any endoscopy probe, including but not limited to the present coated endoscopy probe and present coated ultrasonic lithotripsy probe. The type of vapor deposition used and the particular vapor deposition process parameters employed may be selected taking into account the physical characteristics of the endoscope being coated, such as the material it is composed of, its length, the thickness of the wall between interior and exterior surface, and the size and shape of the opening (such as cylindrical, oval, square, or rectangular in cross-section and linear, tapering, flaring, or complex along its length). By way of non-limiting example, charge may be applied to an interior surface of a workpiece in plasma enhanced chemical vapor deposition in order to produce an interior coating.

While the medical probe has been described with reference to particular embodiments, it will be understood by those skilled in the art that various changes may be made without departing from the intended scope. In addition, many modifications may be made to adapt a particular situation or material to these teachings without departing from the intended scope. In addition, many modifications may be made to adapt a particular situation or material to these teachings without departing from the intended scope.

Therefore, it is intended that the scope not be limited to the particular embodiments disclosed herein, but rather will include all embodiments falling within the scope of the appended claims.

## Claims

1. A medical probe comprising:
a probe body (30) extending along a longitudinal axis and comprising:
an interior surface (38);
an exterior surface (32);
a proximal portion (34) including a proximal end portion adapted for connection to an endoscopy instrument; and
a distal portion (35) including a distal tip (36) and being adapted for insertion into a body of a patient, wherein at least a portion of the exterior surface of the distal portion has a coating of diamond-like carbon or diamond-like carbon derivative;
an aspiration lumen (5) surrounding the probe body; and
an irrigation lumen (4) surrounding the aspiration lumen.

2. The medical probe of claim 1, wherein at least the distal tip has a coating of the diamond-like carbon or diamond-like carbon derivative.

3. The medical probe of claim 2, wherein the coating reduces wear of the distal tip resulting from physical contact with hardened masses.

4. The medical probe of claim 1, wherein the coating of diamond-like carbon or diamond-like carbon derivative has a thickness of from about 1.0 micrometers to about 6.0 micrometers.

5. The medical probe of claim 4, wherein the coating of diamond-like carbon or diamond-like carbon derivative has a thickness of from about 2.0 micrometers to about 4.0 micrometers.

6. The medical probe of claim 1, wherein the coating of diamond-like carbon or diamond-like carbon derivative comprises hydrogenated amorphous carbon containing silicon.

7. The medical probe of claim 1, wherein the coating has a lower reflectivity than an uncoated portion (10) of the medical probe.

8. An endoscope comprising the medical probe according to any of claims 1 to 7.

## Patentansprüche

1. Medizinische Sonde, umfassend:
einen Sondenkörper (30), der sich entlang einer Längsachse erstreckt und umfassend:
eine Innenfläche (38);
eine Außenfläche (32);
einen proximalen Abschnitt (34), der einen proximalen Endabschnitt beinhaltet, der zur Verbindung mit einem Endoskopieinstrument angepasst ist; und
einen distalen Abschnitt (35), der eine distale Spitze (36) beinhaltet und zum Einführen in einen Körper eines Patienten angepasst ist, wobei mindestens ein Teil der Außenfläche des distalen Abschnitts eine Beschichtung aus diamantartigem Kohlenstoff oder einem diamantartigen Kohlenstoffderivat aufweist;
ein Aspirationslumen (5), das den Sondenkörper umgibt; und ein Spüllumen (4), das das Aspirationslumen umgibt.

2. Medizinische Sonde gemäß Anspruch 1, wobei zumindest die distale Spitze eine Beschichtung aus diamantähnlichem Kohlenstoff oder einem diamantähnlichen Kohlenstoffderivat aufweist.

3. Medizinische Sonde gemäß Anspruch 2, wobei die Beschichtung eine Abnutzung der distalen Spitze aufgrund eines physischen Kontakts mit gehärteten Massen verringert.

4. Medizinische Sonde gemäß Anspruch 1, wobei die Beschichtung aus diamantartigem Kohlenstoff oder diamantartigem Kohlenstoffderivat eine Stärke von etwa 1,0 Mikrometer bis etwa 6,0 Mikrometer aufweist.

5. Medizinische Sonde gemäß Anspruch 4, wobei die Beschichtung aus diamantartigem Kohlenstoff oder diamantartigem Kohlenstoffderivat eine Stärke von etwa 2,0 Mikrometern bis etwa 4,0 Mikrometern aufweist.

6. Medizinische Sonde gemäß Anspruch 1, wobei die Beschichtung aus diamantartigem Kohlenstoff oder diamantartigem Kohlenstoffderivat hydrierten amorphen Kohlenstoff umfasst, der Silicium enthält.

7. Medizinische Sonde gemäß Anspruch 1, wobei die Beschichtung ein geringeres Reflexionsvermögen aufweist als ein unbeschichteter Abschnitt (10) der medizinischen Sonde.

8. Endoskop, umfassend die medizinische Sonde gemäß einem der Ansprüche 1 bis 7.

## Revendications

1. Une sonde médicale comprenant :
un corps de sonde (30) s'étendant le long d'un axe longitudinal et comprenant:
une surface intérieure (38) ;
une surface extérieure (32) ;
une partie proximale (34) comprenant une partie d'extrémité proximale adaptée pour être reliée à un instrument d'endoscopie ; et
une partie distale (35) comprenant une pointe distale (36) et étant adaptée pour être insérée dans le corps d'un patient, dans laquelle au moins une partie de la surface extérieure de la partie distale a un revêtement de carbone de type diamant ou de dérivé de carbone de type diamant ;
une lumière d'aspiration (5) entourant le corps de la sonde ; et
une lumière d'irrigation (4) entourant la lumière d'aspiration.

2. La sonde médicale selon la revendication 1, dans laquelle au moins la pointe distale a un revêtement de carbone de type diamant ou de dérivé de carbone de type diamant.

3. La sonde médicale selon la revendication 2, dans laquelle le revêtement réduit l'usure de la pointe distale résultant du contact physique avec des masses durcies.

4. La sonde médicale selon la revendication 1, dans laquelle le revêtement de carbone de type diamant ou de dérivé de carbone de type diamant a une épaisseur d'environ 1,0 micromètre à environ 6,0 micromètres.

5. La sonde médicale selon la revendication 4, dans laquelle le revêtement de carbone de type diamant ou de dérivé de carbone de type diamant a une épaisseur d'environ 2,0 micromètre à environ 4,0 micromètres.

6. La sonde médicale selon la revendication 1, dans laquelle le revêtement de carbone de type diamant ou de dérivé de carbone de type diamant comprend du carbone amorphe hydrogéné contenant du silicium.

7. La sonde médicale selon la revendication 1, dans laquelle le revêtement a une réflectivité inférieure à celle d'une partie non revêtue (10) de la sonde médicale.

8. Endoscope comprenant la sonde médicale selon l'une quelconque des revendications 1 à 7.
